# EUROPEAN PATENT APPLICATION

(11) **EP 4 353 277 A1**
(43) Date of publication of application: **17.04.2024**
(21) Application number: 22864542.0
(22) Date of filing: 30.08.2022
(51) Int. Cl.: A61M 1/36, A61M 1/30

(54) **BLOOD PURIFICATION DEVICE**

(30) Priority: 31.08.2021 JP 2021141827
(71) Applicant: Nikkiso Company Limited, Tokyo 150-6022 (JP)
(72) Inventor: TASHIRO Syu, Makinohara-shi, Shizuoka 421-0496 (JP); SUZUKI Tomohiro, Makinohara-shi, Shizuoka 421-0496 (JP)
(74) Representative: von Hirschhausen, Helge
(86) International application number: PCT/JP2022/032544
(87) International publication number: WO 2023/032960

(57) **Abstract**

To provide a blood purification apparatus configured such that the connection of a chamber member is prevented from being forgotten when blood purification treatment is performed after the switching to a single-needle state. A blood purification apparatus is arbitrarily switchable between a double-needle state and a single-needle state and includes a blood pump 4, a delivery-amount-detecting unit 11 configured to detect the amount of liquid delivery made with the operation of the blood pump 4, a pressure detector 6 configured to detect the pressure in a blood circuit, a chamber member 9 connected to an air-trap chamber 5 in the single-needle state and configured to receive and store the blood in the blood circuit in a blood-collection phase in which blood is collected from the patient and to discharge the stored blood into the blood circuit in a blood-return phase in which the blood is returned to the patient, and a connection-checking unit 12 configured to check whether the chamber member 9 is connected with reference to the amount of liquid delivery detected by the delivery-amount-detecting unit 11 and the pressure detected by the pressure detector 6.

## Description

### Technical Field

The present invention relates to a blood purification apparatus configured to perform blood purification treatment by causing a patient's blood to extracorporeally circulate.

### Background Art

In general, blood purification treatment is performed by using a blood circuit for causing blood collected from a patient to extracorporeally circulate and return into the patient's body. Such a blood circuit basically includes, for example, an arterial blood circuit and a venous blood circuit that are connectable to a dialyzer (a blood purifier) including hollow fiber membranes (blood purification membranes). While the patient's blood is caused to extracorporeally circulate through the blood circuit, the blood is purified with the dialyzer. Thus, blood purification treatment is performed.

A known blood purification apparatus disclosed by PTL 1, for example, is arbitrarily switchable between a double-needle state and a single-needle state. In the double-needle state, an arterial puncture needle and a venous puncture needle are connected to the distal ends of an arterial blood circuit and a venous blood circuit, respectively. In the single-needle state, a single needle is connected to the distal ends of the arterial blood circuit and the venous blood circuit with the aid of a Y-shaped tube.

The above known blood purification apparatus includes a bubble isolation chamber and a single-needle chamber that are connected to each other and between which a single-needle valve is provided. To perform blood purification treatment in the double-needle state, the single-needle valve is closed to allow the blood to extracorporeally circulate. To switch from the double-needle state to the single-needle state and continue the blood purification treatment, the single-needle valve is opened to make the single-needle chamber communicate with the bubble isolation chamber, so that the blood is storable in the single-needle chamber.

### Citation List

### Patent Literature

PTL 1: Japanese Unexamined Patent Application Publication (Translation of PCT Application) No. 2017-520365

### Summary of Invention

### Technical Problem

In the above known blood purification apparatus, to perform blood purification treatment after switching from the double-needle state to the single-needle state, the single-needle valve needs to be controlled to make the single-needle chamber communicate with the bubble isolation chamber. Therefore, the present applicant has examined the possibility of omitting the single-needle valve by connecting a chamber member that is capable of storing blood to the blood circuit for a blood purification treatment to be performed after the switching from the double-needle state to the single-needle state.

Connecting such a chamber member to the blood circuit for a blood purification treatment to be performed after the switching to the single-needle state makes the single-needle valve unnecessary. However, whether the chamber member is connected is undetectable by the blood purification apparatus and needs to be checked only visually by a medical worker. In such a situation, the blood purification treatment in the single-needle state may be performed with the chamber member forgotten to be connected to the blood circuit.

The present invention has been conceived in view of the above circumstances and provides a blood purification apparatus configured such that the connection of a chamber member is prevented from being forgotten when blood purification treatment is performed after the switching to a single-needle state.

### Solution to Problem

A blood purification apparatus according to an embodiment of the present invention is configured to purify a patient's blood by causing the blood to extracorporeally circulate through a blood circuit and a blood purifier, the blood circuit including an arterial blood circuit and a venous blood circuit. The apparatus is arbitrarily switchable between a double-needle state in which an arterial puncture needle and a venous puncture needle are connected to distal ends of the arterial blood circuit and the venous blood circuit, respectively, and a single-needle state in which a single needle is connected to the distal ends of the arterial blood circuit and the venous blood circuit with an aid of a Y-shaped tube. The apparatus includes a blood pump configured to deliver liquid in the blood circuit; a delivery-amount-detecting unit configured to detect an amount of liquid delivery made with operation of the blood pump; a pressure detector configured to detect a pressure in the blood circuit; a chamber member connected in the single-needle state to the blood circuit at a position on a downstream side relative to the blood pump, the chamber member being configured to receive and store the blood in the blood circuit in a blood-collection phase in which blood is collected from the patient and to discharge the stored blood into the blood circuit in a blood-return phase in which the blood is returned to the patient; and a connection-checking unit configured to check whether the chamber member is connected, the checking being performed with reference to the amount of liquid delivery detected by the delivery-amount-detecting unit and the pressure detected by the pressure detector.

### Advantageous Effects of Invention

According to the present invention, whether the chamber member is connected is checked with reference to the amount of liquid delivery detected by the delivery-amount-detecting unit and the pressure detected by the pressure detector. Therefore, when blood purification treatment is performed in the single-needle state, the connection of the chamber member is prevented from being forgotten.

### Brief Description of Drawings

[Fig. 1] Fig. 1 is a schematic diagram of a blood purification apparatus (in a double-needle state) according to an embodiment of the present invention.
[Fig. 2] Fig. 2 is a schematic diagram of the blood purification apparatus (in a single-needle state).
[Fig. 3] Fig. 3 is a schematic diagram of a chamber member included in the blood purification apparatus.
[Fig. 4] Fig. 4 is a schematic diagram of the blood purification apparatus in a state to be established for a connection test.
[Fig. 5] Fig. 5 is a flow chart of a control sequence for a blood purification treatment that is performed by the blood purification apparatus (in the single-needle state).
[Fig. 6] Fig. 6 is a flow chart of a control sequence for the connection test that is performed by the blood purification apparatus (in the single-needle state). Description of Embodiments

An embodiment of the present invention will now be described specifically with reference to the drawings.

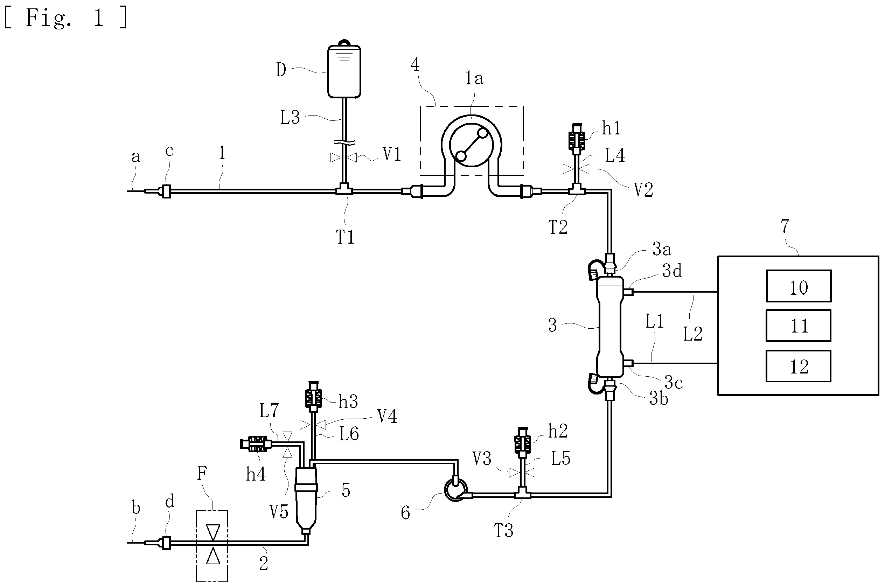

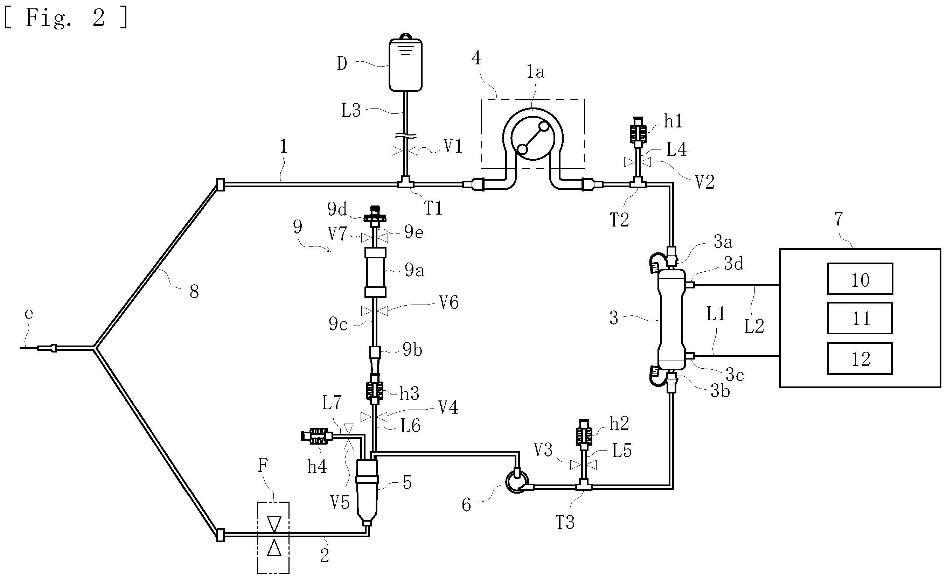

A blood purification apparatus according to the present embodiment is a dialysis apparatus for giving hemodialysis treatment and includes, as illustrated in Figs. 1 and 2, a blood circuit, a dialyzer 3, a blood pump 4, an air-trap chamber 5, a pressure detector 6, and an apparatus body 7. The blood circuit includes an arterial blood circuit 1 and a venous blood circuit 2. The dialyzer 3 serves as a blood purifier. The apparatus body 7 includes a control unit 10, a delivery-amount-detecting unit 11, and a connection-checking unit 12.

The arterial blood circuit 1 is formed of flexible tubes through which predetermined liquid is allowed to flow. The arterial blood circuit 1 is provided at the distal end thereof with a connector c, to which an arterial puncture needle a is attachable. The arterial blood circuit 1 is further provided with a T-shaped tube T1, to which a physiological-saline supply line L3 is connected. The physiological-saline supply line L3 is provided at the distal end thereof with a container D, which is called a saline bag. The physiological-saline supply line L3 is arbitrarily openable and closable with an electromagnetic valve or a pair of forceps, which is not illustrated, so that physiological saline in the container D can be supplied into the blood circuit.

The arterial blood circuit 1 is further provided with a squeezable tube 1a at a halfway position thereof (between the T-shaped tube T1 and a T-shaped tube T2). The squeezable tube 1a is attachable to the blood pump 4. The squeezable tube 1a is to be squeezed in the lengthwise direction thereof while being compressed in the radial direction thereof by the blood pump 4, whereby the liquid in the squeezable tube 1a is caused to flow. The squeezable tube 1a is a flexible tube that is softer and has a greater diameter than the other flexible tubes forming the arterial blood circuit 1.

The blood pump 4 is configured to deliver a priming solution or a patient's blood (liquid) in the blood circuit. The blood pump 4 is a so-called peristaltic pump including a rotor, which is rotatable, and rollers. With the rotation of the rotor, the squeezable tube 1a is squeezable by the rollers in the direction of rotation of the rotor (the lengthwise direction). The squeezing causes the blood in the arterial blood circuit 1 to flow in the direction of rotation of the rotor. Thus, the blood is caused to extracorporeally circulate through the blood circuit 1.

The arterial blood circuit 1 is provided with the T-shaped tube 2 at a position between the squeezable tube 1a and the dialyzer 3. A connection tube L4 extends from the T-shaped tube T2. The venous blood circuit 2 is provided with a T-shaped tube T3 at a position between the dialyzer 3 and the pressure detector 6. A connection tube L5 extends from the T-shaped tube T3. The connection tubes L4 and L5 are provided at the respective distal ends thereof with respective connecting parts h1 and h2, to each of which a distal part or any other part of a syringe is connectable for the injection of desired liquid (a liquid drug or the like). The connection tubes L3 to L5 have respective flow routes that are openable and closable with respective clamps (V1 to V3) (each being an apparatus-side clamp or a blood-circuit-side clamp).

The venous blood circuit 2 is formed of flexible tubes through which predetermined liquid is allowed to flow. The venous blood circuit 2 is provided at the distal end thereof with a connector d, to which a venous puncture needle b is attachable. The air-trap chamber 5 and the pressure detector 6 are connected to the venous blood circuit 2. The venous blood circuit 2 is further provided at a distal part thereof (a part between the connector d and the air-trap chamber 5) with a venous clamp F, which is capable of arbitrarily opening and closing the flow route for the liquid.

The air-trap chamber 5 is configured to remove bubbles from the blood purified by the dialyzer 3 and is connected to the venous blood circuit 2 at a position between the connector d and the pressure detector 6. The air-trap chamber 5 according to the present embodiment is configured to introduce the blood (or the priming solution) from the top thereof and to discharge the blood from the bottom thereof. Before blood purification treatment, the air-trap chamber 5 has no air layer formed at the top of the inside space thereof.

The air-trap chamber 5 has connection tubes L6 and L7 extending from the top thereof. The connection tubes L6 and L7 are provided at the respective distal ends thereof with respective connecting parts h3 and h4, to each of which a distal part or any other part of a syringe is connectable for the injection of desired liquid (a liquid drug or the like) or through each of which air resulting from bubble removal is dischargeable. The connection tubes L6 and L7 have respective flow routes that are openable and closable with respective clamps (V4 and V5) (each being an apparatus-side clamp or a blood-circuit-side clamp).

The pressure detector 6 is connected to a position between the dialyzer 3 and the air-trap chamber 5 and is capable of detecting the pressure in the blood circuit (specifically, the fluid pressure in the venous blood circuit 2). The pressure detector 6 according to the present embodiment includes a liquid-phase part, a gas-phase part, and a diaphragm that separates the liquid-phase part and the gas-phase part from each other. The blood (or the priming solution) that is under extracorporeal circulation is allowed to flow through the liquid-phase part while filling the liquid-phase part. The diaphragm is displaced in correspondence with the fluid pressure of the blood. The pressure of gas (any gas such as air or a predetermined gas) in the gas-phase part changes with the displacement of the diaphragm. By measuring the gas pressure, the pressure in the venous blood circuit 2 (the venous pressure) is detectable.

As described above, in the blood circuit according to the present embodiment, no air layer is formed at the top of the air-trap chamber 5, and the liquid-phase part of the pressure detector 6 is filled with liquid and requires no air layer. Therefore, in a double-needle state and with a chamber member 9 not connected, the blood circuit serves as an airless circuit in which no air layer is to be formed in the flow route for the extracorporeally circulating blood.

The dialyzer 3 has, in a housing thereof, a plurality of hollow fibers each having microscopic holes (pores). The housing has a blood introduction port 3a, a blood delivery port 3b, a dialysate introduction port 3c, and a dialysate delivery port 3d. The proximal end of the arterial blood circuit 1 is connected to the blood introduction port 3a. The proximal end of the venous blood circuit 2 is connected to the blood delivery port 3b. The dialysate introduction port 3c and the dialysate delivery port 3d are connected to a dialysate introduction line L1 and a dialysate discharge line L2, respectively, extending from the apparatus body 7.

The patient's blood introduced into the dialyzer 3 flows inside each of the hollow fiber membranes (blood flow routes) housed therein and is discharged therefrom through the blood delivery port 3b. On the other hand, dialysate introduced into the dialyzer 3 through the dialysate introduction port 3c flows outside the hollow fiber membranes (dialysate flow routes) and is discharged therefrom through the dialysate delivery port 3d. In this process, waste products and the like in the blood flowing in the blood flow routes are caused to permeate through to the dialysate, whereby the blood is purified. Then, the purified blood is returned into the patient's body through the venous blood circuit 2.

To summarize, the dialyzer 3 is connected between the arterial blood circuit 1 and the venous blood circuit 2, and the blood pump 4 is activated with the patient being punctured with the arterial puncture needle a and the venous puncture needle b, whereby the patient's blood is caused to extracorporeally circulate through the blood circuit (the arterial blood circuit 1 and the venous blood circuit 2) and the dialyzer 3 and is returned to the patient after being purified.

In the present embodiment, the double-needle state and a single-needle state are arbitrarily switchable therebetween. In the double-needle state, as illustrated in Fig. 1, the arterial puncture needle a and the venous puncture needle b are connected to the distal ends of the arterial blood circuit 1 and the venous blood circuit 2, respectively. In the single-needle state, as illustrated in Fig. 2, a single needle e is connected to the distal ends of the arterial blood circuit 1 and the venous blood circuit 2 with the aid of a Y-shaped tube 8. The two proximal parts of the Y-shaped tube 8 are connected to the connector c of the arterial blood circuit 1 and the connector d of the venous blood circuit 2. The single needle e is attached to the distal part of the Y-shaped tube 8.

In the blood purification treatment that is performed in the single-needle state, a blood-collection phase and a blood-return phase are executed alternately, whereby the blood is caused to extracorporeally circulate through the blood circuit and is purified with the dialyzer 3. In the blood-collection phase, the patient is punctured with the single needle e, and the blood pump 4 is activated with the venous clamp F closed, whereby the patient's blood is collected through the single needle e. In the blood-return phase, the venous clamp F is opened and the blood pump 4 is stopped, whereby the blood collected in the blood-collection phase is returned to the patient through the single needle e.

In the single-needle state, the chamber member 9 is connected to the connecting part h3 of the air-trap chamber 5. In the blood-collection phase for collecting blood from the patient, the blood in the venous blood circuit 2 is introduced into and stored in the chamber member 9. In the blood-return phase for returning the blood to the patient, the blood stored in the chamber member 9 is discharged into the venous blood circuit 2 for blood return. In the present embodiment, the chamber member 9 is connected to the connecting part h3 attached to the connection tube L6 extending from the top of the air-trap chamber 5. Alternatively, the chamber member 9 may be connected to the connecting part h4 attached to the other connection tube L7.

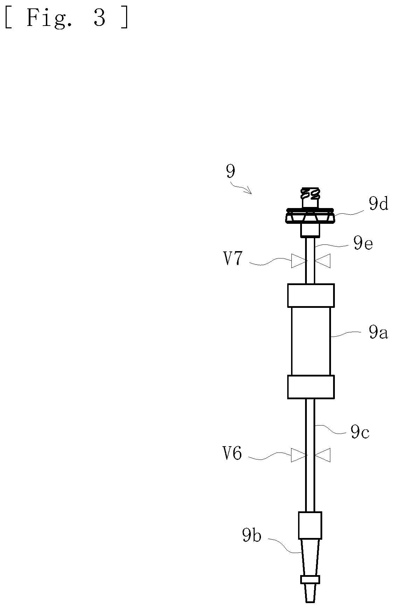

More specifically, as illustrated in Fig. 3, the chamber member 9 includes a chamber 9a, a connecting part 9b, a connecting tube 9c, a hydrophobic filter 9d, and a liquid-level-adjusting tube 9e. The chamber 9a is capable of storing blood thereinside. The connecting part 9b is attachable to and detachable from the connecting part h3 of the air-trap chamber 5. The connecting tube 9c make the connecting part 9b and the chamber 9a communicate with each other, and allows the blood in the air-trap chamber 5 to flow into the chamber 9a when a clamp V6 (an apparatus-side clamp or a blood-circuit-side clamp) is opened. The hydrophobic filter 9d blocks liquid from flowing therethrough but allows gas to flow therethrough. The hydrophobic filter 9d and the chamber 9a are allowed to communicate with each other through the liquid-level-adjusting tube 9e when a clamp V7 (an apparatus-side clamp or a blood-circuit-side clamp) is opened, so that the level of the liquid in the chamber 9a is adjustable.

To perform blood purification treatment in the single-needle state, the connecting part 9b is connected to the connecting part h3 of the air-trap chamber 5, so that the blood is allowed to flow into and be stored in the chamber 9a in the blood-collection phase. In blood purification treatment, the liquid-level-adjusting tube 9e is kept closed with the clamp V7 (an apparatus-side clamp or a blood-circuit-side clamp), whereby the occurrence of pressure release in the blood-collection phase is prevented. To perform blood purification treatment in the double-needle state, the chamber member 9 according to the present embodiment is disconnected. The chamber member 9 is to be connected to the connecting part h3 by a worker at the start of the blood purification treatment to be performed in the single-needle state. That is, the chamber member 9 is not necessary when blood purification treatment is performed in the double-needle state, but is attachable with an arbitrary timing before blood purification treatment is started in the single-needle state.

The apparatus body 7 according to the present embodiment includes the control unit 10, the delivery-amount-detecting unit 11, and the connection-checking unit 12, as well as tubes, pumps, and other relevant elements for causing dialysate and drain liquid to flow. The control unit 10 is a microcomputer or the like that is connected to relevant devices such as actuators and sensors included in the blood purification apparatus. The control unit 10 is capable of controlling various operations for performing blood purification treatment in the double-needle state or the single-needle state.

In the single-needle state, the control unit 10 according to the present embodiment closes the venous clamp F and activates the blood pump 4, thereby executing blood collection in the blood-collection phase. When the pressure (venous pressure) detected by the pressure detector 6 reaches a preset upper-limit pressure, the control unit 10 switches to the blood-return phase, in which the control unit 10 opens the venous clamp F and stops the blood pump 4, thereby executing blood return in the blood-return phase. When the pressure (venous pressure) detected by the pressure detector 6 reaches a preset lower-limit pressure, the control unit 10 switches to the blood-collection phase. Thus, the blood-collection phase and the blood-return phase are executed alternately, whereby the blood is caused to extracorporeally circulate through the blood circuit.

The delivery-amount-detecting unit 11 is a microcomputer or the like that is electrically connected to the blood pump 4. The delivery-amount-detecting unit 11 is configured to detect the amount of liquid delivery made with the operation of the blood pump 4. More specifically, the delivery-amount-detecting unit 11 according to the present embodiment is capable of detecting the amount of delivery (the stroke volume) of liquid, such as blood or the priming solution, with reference to the number of revolutions of the rotor of the blood pump 4. The amount of liquid delivery may alternatively be detected with reference to any parameter other than the number of revolutions of the rotor of the blood pump 4.

The connection-checking unit 12 is a microcomputer or the like that is electrically connected to the delivery-amount-detecting unit 11 and to the pressure detector 6. The connection-checking unit 12 is configured to check whether the chamber member 9 is connected, with reference to the amount of liquid delivery detected by the delivery-amount-detecting unit 11 and the pressure (venous pressure) detected by the pressure detector 6. More specifically, in the single-needle state, the connection-checking unit 12 according to the present embodiment checks whether the amount of liquid delivery detected when the upper-limit pressure at which the blood-collection phase is switched to the blood-return phase is reached exceeds a preset reference value. If the amount of liquid delivery does not exceed the reference value, it is determined that the chamber member 9 is not connected (forgotten to be connected).

In the present embodiment, the checking by the connection-checking unit 12 of whether the chamber member 9 is connected is performed not only during the blood purification treatment that is performed in the single-needle state as described above but also in a connection test that is conducted with pressure application before blood purification treatment. The connection test is to be conducted before blood purification treatment so that whether the connection of the chamber member 9 is normal is checked, with the application of a pressure (fluid pressure) to the tubes in the apparatus body 7 and to the blood circuit. In the present embodiment, the checking by the connection-checking unit 12 of whether the chamber member 9 is connected is performed in addition to various connection tests.

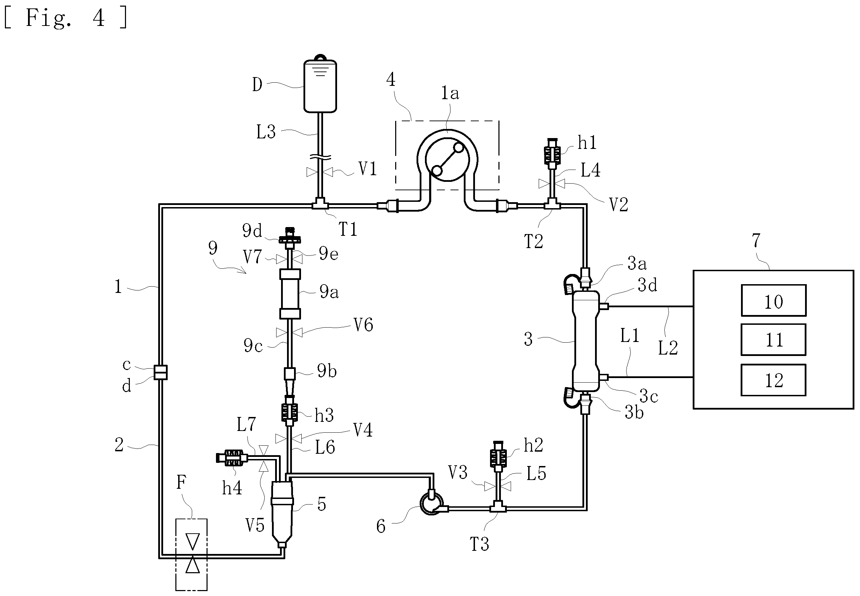

More specifically, before blood purification treatment is started, as illustrated in Fig. 4, the connector c of the arterial blood circuit 1 and the connector d of the venous blood circuit 2 are connected to each other to establish a closed circuit, and the blood pump 4 is activated to cause the physiological saline (priming solution) in the container D to circulate through the closed circuit, whereby a priming process is performed. Before or after the priming process, whether the chamber member 9 is connected can be checked.

In this case, the blood pump 4 is activated with the venous clamp F closed. Furthermore, when the amount of delivery of the physiological saline (priming solution) with the operation of the blood pump 4 reaches a predetermined value, the connection-checking unit 12 checks whether the amount of pressure rise (the amount of venous-pressure rise) detected by the pressure detector 6 is greater than a preset reference value. If the amount of pressure rise is greater than the reference value, it is determined that the chamber member 9 is not connected. For example, if the reference value is set to 4.0 mL, the initial value may be set to 4.0 mL, with the variable range being 0 (not monitored) to 40.0 mL. In this case, the stroke volume in the blood circuit with the chamber member 9 not connected is 3.0 mL, and the stroke volume in the blood circuit with the chamber member 9 connected or in a normal blood circuit is about 4.5 to 6.0 mL. Therefore, setting the reference value to 4.0 mL enables the detection of the state where the chamber member 9 is not connected, and suppresses the occurrence of a wrong warning under any other situation. Considering possible complexity in providing such information, the range of the preset value may preferably be 0 to 40.0 mL, with 0 mL being the lower limit at which the value is not monitored substantially, and with 40.0 mL being the upper limit including some allowance. More preferably, the range may be changed arbitrarily in correspondence with the phase-switching pressures (the upper-limit pressure and the lower-limit pressure) or the venous pressure of the patient.

The above description relates to a case where whether the chamber member 9 is connected is checked with reference to the pressure rise (the amount of venous-pressure rise) in the blood circuit that is detected when the amount of liquid delivery with the operation of the blood pump 4 reaches a predetermined value. Alternatively, whether the chamber member 9 is connected may be checked by operating the blood pump 4 until the pressure rise (venous-pressure rise) in the blood circuit reaches a predetermined value and detecting the amount of liquid delivery when the predetermined value is reached.

In the present embodiment, whether the chamber member 9 is connected is checked with the blood circuit filled with the priming solution (physiological saline or dialysate). Alternatively, whether the chamber member 9 is connected may be checked with the blood circuit supplied with air. In the above embodiment, the priming solution that is caused to flow through the blood circuit is physiological saline. Alternatively, the priming solution may be dialysate supplied from the outside of the blood circuit, for example, from the apparatus body 7 or the like. In the latter case, whether the chamber member 9 is connected is checked by detecting the rise of the fluid pressure that occurs with the supply of the dialysate (priming solution), and the venous clamp F does not need to be closed.

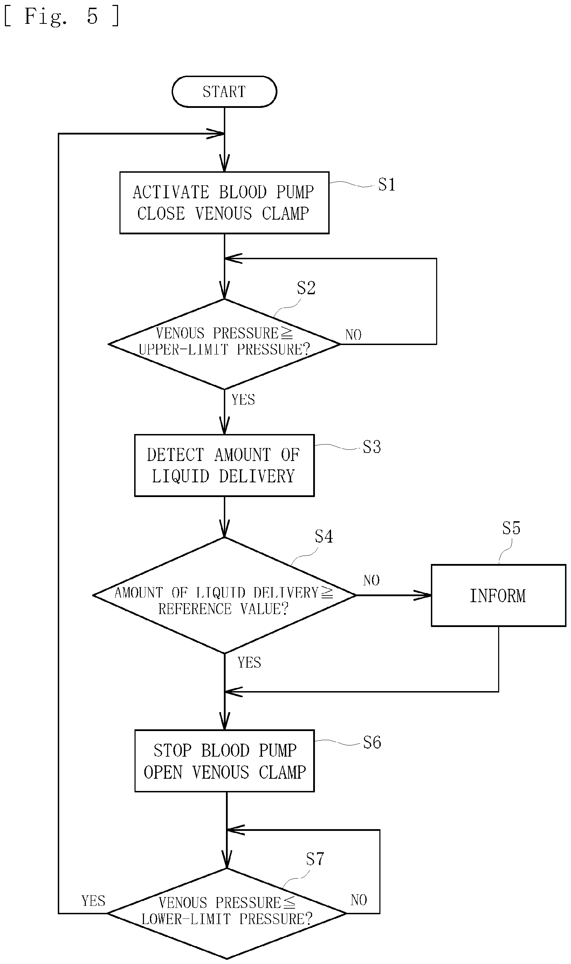

Now, a control sequence for the blood purification treatment that is performed in the single-needle state by the blood purification apparatus according to the present embodiment will be described with reference to the schematic diagram illustrated in Fig. 2 and the flow chart illustrated in Fig. 5.

First, the patient is punctured with the single needle e, and the blood pump 4 is then activated with the venous clamp F closed (S1). Subsequently, whether the pressure (venous pressure) detected by the pressure detector 6 has reached the upper-limit pressure is checked (S2).

If it is determined in S2 that the pressure (venous pressure) detected by the pressure detector 6 has reached the upper-limit pressure, the amount of liquid delivery is detected with reference to the amount of operation of the blood pump 4 (S3). Then, whether the detected amount of liquid delivery is greater than a preset reference value is checked (S4). If it is determined that the amount of liquid delivery is not greater than the preset reference value (the amount of liquid delivery is smaller than the reference value), an informing device included in the apparatus body 7 or the like provides information in S5 that the chamber member 9 is not connected, and the sequence proceeds to S6. On the other hand, if it is determined that the amount of liquid delivery is smaller than the preset reference value (the amount of liquid delivery is greater than the reference value), the provision of the information in S5 is skipped, and the sequence proceeds to S6.

Thus, the blood-collection phase is ended, and the blood-return phase is started. In the blood-return phase, with the venous clamp F opened, the blood pump 4 is stopped (S6). Then, whether the pressure (venous pressure) detected by the pressure detector 6 has reached the lower-limit pressure is checked (S7). If it is determined in S7 that the pressure (venous pressure) detected by the pressure detector 6 has reached the lower-limit pressure, the sequence returns to S1, where the blood-collection phase is started again. Through the above steps, the blood-collection phase and the blood-return phase are executed alternately. Thus, the patient's blood is purified with the dialyzer 3 while being caused to extracorporeally circulate through the blood circuit.

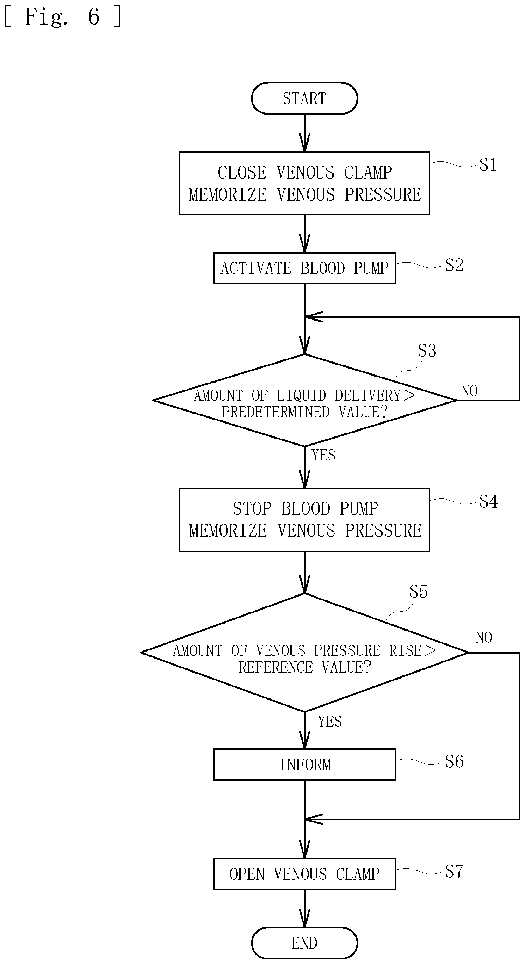

Now, a control sequence for the connection test that is performed in the single-needle state by the blood purification apparatus according to the present embodiment will be described with reference to the schematic diagram illustrated in Fig. 4 and the flow chart illustrated in Fig. 6.

First, the connector c of the arterial blood circuit 1 and the connector d of the venous blood circuit 2 are connected to each other to establish a closed circuit. Then, the venous clamp F is closed, and the pressure (venous pressure) detected by the pressure detector 6 is memorized (S1).

Subsequently, in S2, the blood pump 4 is activated. Then, in S3, whether the amount of liquid delivery with the operation of the blood pump 4 is greater than a predetermined value is checked. If it is determined in S3 that the amount of liquid delivery with the operation of the blood pump 4 is greater than the predetermined value, the blood pump 4 is stopped, and the pressure (venous pressure) detected by the pressure detector 6 is memorized (S4). Accordingly, the amount of pressure rise is obtained from the pressure (venous pressure) memorized in S1 and the pressure (venous pressure) memorized in S4.

Furthermore, whether the amount of pressure rise obtained is greater than a preset reference value is checked (S5). If it is determined that the amount of pressure rise is greater than the reference value, the informing device included in the apparatus body 7 or the like provides information in S6 that the chamber member 9 is not connected, and the sequence proceeds to S7. On the other hand, if it is determined that the amount of pressure rise is not smaller than the reference value (the amount of pressure rise is not greater than the reference value), the provision of the information in S6 is skipped, and the sequence proceeds to S7. In S7, the venous clamp F is opened. Thus, the connection test is ended.

According to the present embodiment, whether the chamber member 9 is connected is checked with reference to the amount of liquid delivery detected by the delivery-amount-detecting unit 11 and the pressure (in the present embodiment, the venous pressure) detected by the pressure detector 6. Therefore, when blood purification treatment is performed in the single-needle state, the connection of the chamber member 9 is prevented from being forgotten. In particular, in the present embodiment, if it is determined by the connection-checking unit 12 that the chamber member 9 is not connected, the informing device provides information. Therefore, medical workers or any other personnel are assuredly informed that the connection of the chamber member 9 is forgotten.

According to the present embodiment, the blood purification apparatus includes the venous clamp F capable of opening and closing the flow route at the distal part of the venous blood circuit 2, and the control unit 10. The control unit 10 is configured to control switching between the blood-collection phase and the blood-return phase in the single-needle state in such a manner as to execute the blood-collection phase for blood collection by activating the blood pump 4 with the venous clamp F closed, to switch to the blood-return phase when the pressure detected by the pressure detector 6 reaches a preset upper-limit pressure, to execute the blood-return phase for blood return by opening the venous clamp F and stopping the blood pump 4, and to switch to the blood-collection phase when the pressure detected by the pressure detector 6 reaches a preset lower-limit pressure. Furthermore, the connection-checking unit 12 checks whether the chamber member 9 is connected by checking whether the amount of liquid delivery detected when the upper-limit pressure at which the blood-collection phase is switched to the blood-return phase is reached exceeds a preset reference value. Thus, whether the chamber member 9 is connected is checked during the blood purification treatment performed in the single-needle state.

Furthermore, the blood purification apparatus includes the air-trap chamber 5 that is connected to the venous blood circuit 2 and to and from which the chamber member 9 is attachable and detachable. Therefore, the chamber member 9 is connectable arbitrarily only when blood purification treatment is performed in the single-needle state. In particular, the air-trap chamber 5 is provided with the connection tube L6 extending from the top thereof, and the chamber member 9 is connectable to the connection tube L6 with an arbitrary timing. That is, the connection tube L6 intended for another use, such as the injection of a drug, in blood purification treatment can be utilized to connect the chamber member 9. Alternatively, the chamber member 9 may be connected to the connection tube L7 with an arbitrary timing.

Furthermore, according to the present embodiment, the checking by the connection-checking unit 12 of whether the chamber member 9 is connected is performed at the start of the blood purification treatment to be performed in the single-needle state. Therefore, the blood purification treatment in the single-needle state is assuredly prevented from being started with the connection of the chamber member 9 being forgotten. Furthermore, the checking by the connection-checking unit 12 of whether the chamber member 9 is connected may be performed in the connection test that is conducted with pressure application before blood purification treatment. In such a case, when blood purification treatment is performed in the single-needle state after the connection test, the connection of the chamber member 9 is prevented from being forgotten.

Furthermore, the blood circuit to be applied to the present embodiment is an airless circuit in which no air layer is to be formed in the flow route through which the blood extracorporeally circulates in the double-needle state. Therefore, when blood purification treatment is performed in the double-needle state, blood is prevented from coming into contact with air in any air layer that may be formed in the blood circuit. Moreover, the blood circuit does not need to be exchanged with another one at the switching from the double-needle state to the single-needle state.

Specifically, when an airless circuit is used in the blood purification treatment performed in the single-needle state, substantially no air layer is formed in the blood flow route. In such a case, the known blood circuit is incapable of storing blood of a satisfactory amount in the blood-collection phase, which may lower the efficiency of dialysis. Hence, in the known art, not only an airless circuit but also a normal blood circuit intended to perform blood purification treatment in the single-needle state needs to be prepared.

Furthermore, in the known art, to continue a blood purification treatment started in the double-needle state by using an airless circuit but then switched to the single-needle state, the airless circuit needs to be exchanged with a normal blood circuit. That is, the blood remaining in the airless circuit needs to be returned to the patient before the airless circuit is exchanged with the normal blood circuit. Such an exchanging process is annoying. Moreover, after the blood purification treatment, both the normal blood circuit and the airless circuit are to be disposed of. Accordingly, the number of blood circuits to be disposed of increases, which is disadvantageous.

In contrast, according to the present embodiment, the chamber member 9 is connected when blood purification treatment is performed in the single-needle state. Therefore, while the effect produced by the airless circuit is maintained for the blood purification treatment in the double-needle state, favorable performance of the blood purification treatment in the single-needle state is achieved.

While an embodiment has been described above, the present invention is not limited thereto. For example, the chamber member 9 may be connected not only to the top of the air-trap chamber 5 but also to another position of the blood circuit that is on the downstream side relative to the blood pump 4 (any position such as the connection tube L4 located between the blood pump 4 and the dialyzer 3, or the connection tube L5 located between the dialyzer 3 and the pressure detector 6).

Furthermore, the connection-checking unit 12 according to the above embodiment checks whether the chamber member 9 is connected by checking whether the amount of liquid delivery detected when the upper-limit pressure at which the blood-collection phase is switched to the blood-return phase is reached exceeds a preset reference value. Alternatively, the connection-checking unit 12 may check whether the chamber member 9 is connected by checking whether the amount of liquid delivery detected when a predetermined pressure (fluid pressure) different from the upper-limit pressure is reached exceeds a reference value. That is, the connection-checking unit 12 may check whether the chamber member 9 is connected by checking whether the pressure (fluid pressure) detected by the pressure detector 6 when the amount of liquid delivery reaches a predetermined value exceeds a predetermined value.

While the blood circuit according to the above embodiment is an airless circuit, the blood circuit may alternatively be a normal blood circuit (a blood circuit in which any air layer is to be formed) that is not an airless circuit. Moreover, if it is determined by the connection-checking unit 12 that the chamber member 9 is not connected, the blood purification treatment may be automatically stopped, for example, instead of providing information. The information to be provided when the chamber member 9 is not connected may be in any form, such as information to be displayed on a monitor, information to be outputted from a speaker, or information to be provided by turning on or blinking a lamp.

### Industrial Applicability

The present invention is also applicable to any blood purification apparatus having a different appearance, additional functions, or the like within the spirit of the present invention.

### Reference Signs List

- 1: arterial blood circuit
- 1a: squeezable tube
- 2: venous blood circuit
- 3: dialyzer (blood purifier)
- 3a: blood introduction port
- 3b: blood delivery port
- 3c: dialysate introduction port
- 3d: dialysate delivery port
- 4: blood pump
- 5: air-trap chamber
- 6: pressure detector
- 7: apparatus body
- 8: Y-shaped tube
- 9: chamber member
- 9a: chamber
- 9b: connecting part
- 9c: connecting tube
- 9d: hydrophobic filter
- 9e: liquid-level-adjusting tube
- 10: control unit
- 11: delivery-amount-detecting unit
- 12: connection-checking unit
- a: arterial puncture needle
- b: venous puncture needle
- c, d: connector
- e: single needle
- D: container
- T1 to T3: T-shaped tube
- F: venous clamp
- L3: physiological-saline supply line
- L4 to L7: connection tube

## Claims

1. A blood purification apparatus configured to purify a patient's blood by causing the blood to extracorporeally circulate through a blood circuit and a blood purifier, the blood circuit including an arterial blood circuit and a venous blood circuit, the apparatus being arbitrarily switchable between a double-needle state in which an arterial puncture needle and a venous puncture needle are connected to distal ends of the arterial blood circuit and the venous blood circuit, respectively, and a single-needle state in which a single needle is connected to the distal ends of the arterial blood circuit and the venous blood circuit with an aid of a Y-shaped tube, the apparatus comprising:
a blood pump configured to deliver liquid in the blood circuit;
a delivery-amount-detecting unit configured to detect an amount of liquid delivery made with operation of the blood pump;
a pressure detector configured to detect a pressure in the blood circuit;
a chamber member connected in the single-needle state to the blood circuit at a position on a downstream side relative to the blood pump, the chamber member being configured to receive and store the blood in the blood circuit in a blood-collection phase in which blood is collected from the patient and to discharge the stored blood into the blood circuit in a blood-return phase in which the blood is returned to the patient; and
a connection-checking unit configured to check whether the chamber member is connected, the checking being performed with reference to the amount of liquid delivery detected by the delivery-amount-detecting unit and the pressure detected by the pressure detector.

2. The blood purification apparatus according to Claim 1, further comprising:
a venous clamp capable of opening and closing a flow route at a distal part of the venous blood circuit; and
a control unit configured to control switching between the blood-collection phase and the blood-return phase in the single-needle state in such a manner as to execute the blood-collection phase for blood collection by activating the blood pump with the venous clamp closed, to switch to the blood-return phase when the pressure detected by the pressure detector reaches a preset upper-limit pressure, to execute the blood-return phase for blood return by opening the venous clamp and stopping the blood pump, and to switch to the blood-collection phase when the pressure detected by the pressure detector reaches a preset lower-limit pressure,
wherein the connection-checking unit checks whether the chamber member is connected by checking whether the amount of liquid delivery detected when the upper-limit pressure at which the blood-collection phase is switched to the blood-return phase is reached exceeds a preset reference value.

3. The blood purification apparatus according to Claim 1 or 2, further comprising an air-trap chamber that is connected to the venous blood circuit and to and from which the chamber member is attachable and detachable.

4. The blood purification apparatus according to Claim 3, wherein the air-trap chamber is provided with a connection tube extending from a top of the air-trap chamber, and the chamber member is connectable to the connection tube with an arbitrary timing.

5. The blood purification apparatus according to any of Claims 1 to 4, wherein the checking by the connection-checking unit of whether the chamber member is connected is performed at a start of a blood purification treatment to be performed in the single-needle state.

6. The blood purification apparatus according to any of Claims 1 to 5, wherein the checking by the connection-checking unit of whether the chamber member is connected is performed in a connection test that is conducted with pressure application before blood purification treatment.

7. The blood purification apparatus according to any of Claims 1 to 6, wherein the blood circuit is an airless circuit in which no air layer is to be formed in a flow route through which the blood extracorporeally circulates in the double-needle state.
